(19) Europäisches Patentamt
European Patent Office
Office européen des brevets

(11) Publication number: 0 007 205 A1

# (12) EUROPEAN PATENT APPLICATION

(21) Application number: 79301280.8

(22) Date of filing: 02.07.79

(51) Int. Cl.³: C 07 C 91/16
A 61 K 31/135, A 61 K 31/165
A 61 K 31/215, C 07 C 91/30
C 07 C 91/34, C 07 C 93/26
C 07 C 101/42, C 07 C 103/28
C 07 C 103/76

(30) Priority: 03.07.78 US 921867

(43) Date of publication of application: 23.01.80 Bulletin 80/2

(84) Designated Contracting States: DE GB IT NL SE

(71) Applicant: ELI LILLY AND COMPANY
307, East McCarty Street
Indianapolis Indiana(US)

(72) Inventor: Mills, Jack
7902, Timberhill Drive
Indianapolis, Indiana 46217(US)

(72) Inventor: Schmiegel, Klaus Kurt
4507 Staughton Drive
Indianapolis, Indiana 46226(US)

(72) Inventor: Tuttle, Ronald Ralph
4540 Berkshire Road
Indianapolis, Indiana 46226(US)

(74) Representative: McVey, Kenneth William Henry et al,
Erl Wood Manor
Windlesham Surrey, GU20 6PH(GB)

## (54) Optically active phenethanolamines, their formulations, use and preparation.

(57) Compounds of the formula (I):

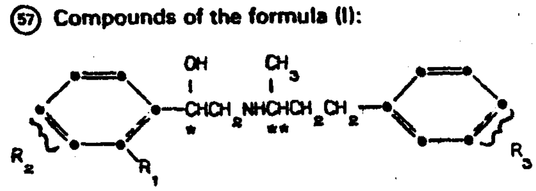

(I)

wherein:

$R_1$ is hydrogen or fluorine;

$R_2$ is hydrogen or hydroxy;

$R_3$ is hydrogen, hydroxy, fluorine, aminocarbonyl, methylaminocarbonyl, methoxycarbonyl or acetoxy;

C and C both are asymmetric carbon atoms having
* and **
the R absolute stereochemical configuration;

with the limitation that at least one of $R_1$ and $R_2$ is hydrogen;

or pharmaceutically acceptable salts thereof are extremely potent inotropic agents.

EP 0 007 205 A1

# OPTICALLY ACTIVE PHENETHANOLAMINES, THEIR FORMULATIONS, USE AND PREPARATION

This invention relates to certain extraordinarily potent inotropic agents, to pharmaceutical formulations containing those compounds, to the use of the compounds in conditioning cardiovascular systems and to their preparation.

Some of the most extensive efforts in basic research have been in the area of β-phenylethylamine derivatives, many of which are catecholamines. Such compounds are known to have a variety of pharmacological activities. For example, the naturally occurring catecholamine epinephrine is a potent sympathomimetic drug and a powerful cardiac stimulant. However, epinephrine has found only limited utility in restoring cardiac function in cases of cardiac arrest, even though it is one of the most potent vasopressor drugs known, and greatly increases systolic blood pressure. Like most drugs of its class, the use of epinephrine is limited due to its undesirable side effects, which include fear, anxiety, tremor, tenseness, throbbing headache, increased blood pressure, dizziness, respiratory difficulty and palpitation as well as its short duration of action.

Other drugs similar to epinephrine both in structure and physiological properties include two other natural catecholamines, norepinephrine and dopamine, as well as isoproterenol, a synthetic drug. Such sympathomimetic agents currently are used to

restore contractility in cardiogenic shock. However, like other drugs of this class, these agents have side effects which are life threatening, thus greatly inhibiting their clinical usefulness.

Certain catecholamines have been found to exert a positive inotropic effect on heart muscle without the detrimental side effect of significant increase in heart rate or distortion of cardiac rhythm or blood pressure. U.S. Patent No. 3,987,200 discloses a method of increasing cardiac contractility utilizing compounds such as dl-3,4-dihydroxy-N-[3-(4-hydroxyphenyl)-1-methyl-n-propyl]-β-phenethylamine hydrochloride, now generically referred to as dobutamine. Dobutamine is well suited for treating myocardial infarction with failure, congestive heart failure, cardiac by-pass surgery and traumatic surgery. However, like most other catechol derivatives, it is rapidly inactivated in a biological system by the actions of catechol-O-methyl transferase. Dobutamine and its related drugs thus are restricted to intravenous infusion in hospitalized patients.

Even though numerous compounds which have inotropic properties are known, the use of such compounds is severely limited due to their accompanying undesirable side-effects and rapid metabolic inactivation. In many cases the treatment of congestive heart failure can only be effectively accomplished with the use of digitalis and allied cardiac glycosides. The main pharmacodynamic activity of digitalis is its

ability to increase the force of myocardial contraction. Digitalis is thus widely used in the treatment of cardiac failure. Whilst digitalis and its related glycosides are widely used, they remain some of the most dangerous drugs employed. All digitalis-type preparations are toxic at high dose levels. Digitalis toxicity in the heart can be lethal, most of digitalis poisoning being due to the cumulative effect of maintenance doses taken over a relatively long period of time, or from the use of a large dose in the treatment of severe atrial arrhythmias.

The Applicants have now discovered that, surprisingly, the positive inotropic activity of a specific class of phenethanolamines resides almost exclusively in one optical isomer, whilst other non-target biological effects are attributable to the other optically active isomers. This invention therefore provides a means of treating conditions of depressed cardiac contractility utilizing optically active phenethanolamine derivatives which are positive inotropic agents substantially devoid of other biological properties.

An object of this invention therefore is the provision of novel compounds which are positive inotropic agents but which cause little or no undesirable side effects. A further object is to provide a method for treating heart failure utilizing compounds which are direct acting, have an immediate

onset of activity, and are not rapidly inactivated when administered to a biological system. The compounds of this invention provide a low risk of arrhythmia and do not cause vasoconstriction. A further object of this invention is to provide an acceptable alternative to digitalis therapy.

The invention is concerned with certain phenethanolamine derivatives which are particularly useful in the treatment of conditions of depressed cardiac contractility, to pharmaceutical formulations containing such phenethanolamines, and to a method of treating conditions of depressed cardiac contractility, i.e. inadequate pumping function, as well as a method for physically conditioning a biological system. More particularly, the invention provides optically active positive inotropic agents which are phenethanolamine derivatives having the general structural formula (I)

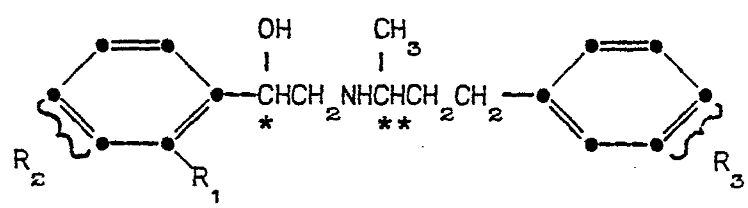
(I)

wherein:

$R_1$ is hydrogen or fluorine;

$R_2$ is hydrogen or hydroxy;

$R_3$ is hydrogen, hydroxy, fluorine, aminocarbonyl, methylaminocarbonyl, methoxycarbonyl or acetoxy;

$C^*$ and $C^{**}$ both are asymmetric carbon atoms having the R absolute stereochemical configuration;

with the limitation that at least one of $R_1$ and $R_2$ is hydrogen; and

the pharmaceutically-acceptable salts thereof.

A preferred group of compounds provided by this invention have the above formula wherein $R_3$ is hydrogen, hydroxy, fluorine, aminocarbonyl, methylaminocarbonyl or methoxycarbonyl.

Another preferred group of compounds have the above formula wherein $R_2$ is hydroxy and $R_3$ is hydroxy, aminocarbonyl or methoxycarbonyl.

The most preferred compounds of the invention have the above formula wherein:

$R_1$ is hydrogen;

$R_2$ is hydrogen or 4-hydroxy; and

$R_3$ is 4-hydroxy or 4-aminocarbonyl; and the pharmaceutically acceptable salts thereof.

A further embodiment of this invention is a pharmaceutical composition comprising a phenethanolamine of formula (I), or a pharmaceutically acceptable salt thereof, associated with at least one pharmaceutical diluent or carrier therefor. An especially preferred formulation comprises the compound R,R-N-[2-(4-hydroxyphenyl)-2-hydroxyethyl]-1-methyl-3-(4-hydroxyphenyl)propylaminium chloride in combination with any of a number of pharmaceutically acceptable diluents.

Such formulation is particularly well suited to administration in a buccal dosage form or via the transdermal, oral or rectal routes.

Yet another embodiment of this invention is a method for physically conditioning a biological system by administering an effective dose of a compound having the above general formula. According to such method, a subject can experience the beneficial effects of physical exercise by the administration of an effective dose of a compound of this invention and thereby obviate the actual physical work.

The compounds provided by this invention are defined by the above general formula. While such compounds are referred to generally as phenethanolamines, they will be named systematically as propylamine derivatives, specifically as N-(2-phenyl or substituted phenyl-2-hydroxyethyl)-1-methyl-3-(substituted phenyl)propylamines. As shown in the above formula, the compounds of this invention have two asymmetric carbon atoms, defined in the formula as $C_*$ and $C_{**}$. The compounds provided by this invention are those optically active isomers having the above formula in which both asymmetric carbon atoms are of the R absolute stereochemical configuration. A complete discussion of absolute stereochemical configuration and nomenclature is presented by Cahn <u>et al</u>. in <u>Experientia</u>, Vol. XII, pp. 81-124 (1956). The stereochemical configuration of the two asymmetric carbon atoms in the compounds of this invention are

designated first when naming the compounds provided herein. Accordingly, a typical compound of this invention will be named as: R,R-N-[2-(4-hydroxyphenyl)-2-hydroxyethyl]-1-methyl-3-(4-aminocarbonylphenyl)propylamine.

The compounds of this invention can be prepared by any of a number of art recognized synthetic routes utilizing readily available starting materials. One such process comprises coupling an optically active mandelic acid derivative with an optically active 1-methyl-3-(substituted phenyl)propylamine to provide an amide, which upon subsequent reduction provides an amine of this invention.

According to one aspect of the invention there is provided a process for preparing a compound of formula (I) which comprises reducing a compound of formula:

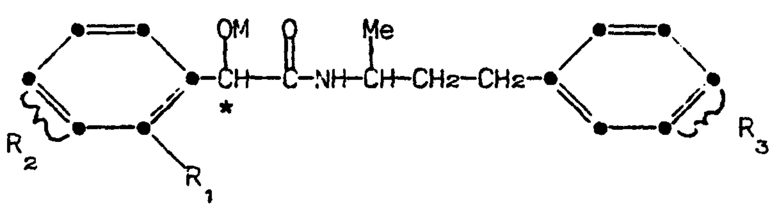

where $R_1$, $R_2$ and $R_3$ are as defined above, and M is hydrogen or a protecting group, followed in the case where M is not hydrogen by removal of the protecting group by hydrolysis.

The intermediate amide can be prepared by reaction of a phenylpropylamine with a hydroxy protected mandelic acid halide, or preferably by direct

coupling reactions utilizing art recognized peptide coupling agents such as N,N'-dicyclohexylcarbodiimide (DCC), carbonyldiimidazole, N-ethyl-5-phenylisoxazolinium-3'-sulfonate and N-ethoxycarbonyl-2-ethoxy-1,2-dihydroquinoline, commonly referred to as EEDQ. Accelerators such as 1-hydroxybenzotriazole can be utilized in the coupling reaction if desired. In a typical coupling reaction, an optically active mandelic acid such as R-2-(3-hydroxyphenyl)-2-hydroxyacetic acid is mixed with approximately equimolar quantities of an optically active phenylpropylamine such as R-1-methyl-3-(3-fluorophenyl)propylamine, and a coupling agent such as DCC or EEDQ.

The reaction generally is carried out in a solvent such as benzene, dimethylformamide, dichloromethane, or the like, and at a temperature of about -30°C. to about 100°C. Under such conditions the reaction routinely is substantially complete within from about 2 to about 48 hours. The product, an amide, is readily isolated by simply filtering the reaction mixture and then removing the reaction solvent by evaporation.

The amide so formed next is reduced at the amide functionality to afford a compound of this invention. Such reduction can be accomplished by reaction of the amide with a common reducing agent such as diborane. For example, an amide such as R,R-N-[2-(3-hydroxyphenyl)-2-hydroxy-1-oxoethyl]-1-methyl-3-(3-fluorophenyl)propylamine can be reacted

with an equimolar quantity or an excess of diborane in an organic solvent such as tetrahydrofuran, benzene, toluene, diethyl ether, dichloromethane or the like. The reaction typically is carried out at a temperature of about 0°C. to about 60°C., and normally is complete within about 6 to about 72 hours. Any excess diborane can be decomposed by the addition of an alcohol such as methanol or ethanol and an acid such as hydrochloric acid. The product amine is readily recovered by removal of the reaction solvent by evaporation, and further purification of the product can be accomplished by standard methods such as chromatography, crystallization, salt formation, and the like.

The compounds of this invention can alternatively be prepared directly by the reaction of a styrene oxide, preferably an optically active styrene oxide, with an optically active 1-methyl-3-phenylpropylamine. For instance, a styrene oxide such as R-2-fluorostyrene oxide can be reacted with about an equimolar quantity of an amine such as R-1-methyl-3-(4-methylaminocarbonylphenyl)propylamine in an inert organic solvent such as ethanol, dioxane, toluene or dimethylformamide. The reaction generally is carried out at a temperature of about 50 to about 120°C., and at such temperature the reaction routinely is complete in about 6 to 10 hours. The product, an optically active compound of this invention, is readily isolated by simply removing the reaction solvent, and further purification may be accomplished if desired by normal methods, including chromatography and crystallization.

This reaction can be schematically illustrated by the following reaction schedule:

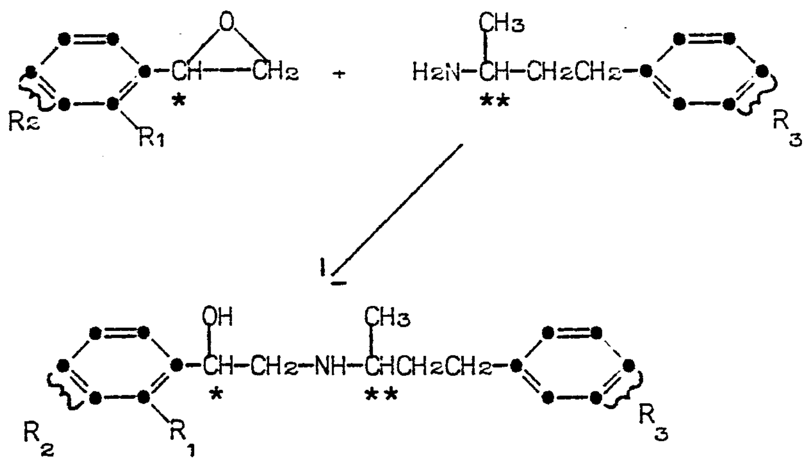

(IV)

where $R_1$, $R_2$ and $R_3$ are as previously defined.

The optically active amines of formula (IV) are novel and are provided in a further aspect of the invention.

They can be prepared from the corresponding ketones of formula:

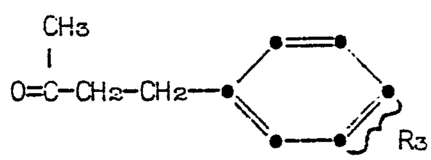

by reaction with (+)-α-methylbenzylamine in the presence of an acid such as p-toluenesulfonic acid to provide the corresponding R-N-(α-methylbenzyl)-1-methyl-3-(($R_3$)phenyl)propylimine. Reduction of this imine with Raney nickel gives

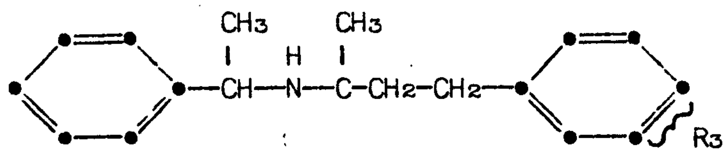

The above amine is salified, for example with HCl, and the salt purified by repeated crystallization from a suitable organic solvent. The optically active R,R-N-(α-methylbenzyl)-1-methyl-3-(($R_3$)-phenyl)propylaminium salt thus obtained can then be hydrogenated with palladium on charcoal to remove the α-methylbenzyl group and liberate the amine of formula IV in salt form.

Yet another method for preparing compounds of this invention comprises reacting an optically active R-1-methyl-3-(substituted phenyl)propylamine with a benzoylmethyl alkylating agent such as a benzoylmethyl halide. Such alkylation reaction affords an N-benzoylmethyl-3-phenylpropylamine derivative. The latter compound is reduced conventionally for instance by catalytic hydrogenation to afford a compound of this invention. For example, reaction of a compound such as R-1-methyl-3-(4-hydroxyphenyl)-propylamine with an alkylating agent such as 3-hydroxybenzoylmethyl bromide, following normal alkylation procedures, provides R-N-[2-(3-hydroxyphenyl)-2-oxoethyl]-1-methyl-3-(4-hydroxyphenyl)-propylamine. Reduction of the oxo group

of the latter compound, for instance by reaction with sodium borohydride or by catalytic hydrogenation, provides a mixture of R,R-N-[2-(3-hydroxyphenyl)-2-hydroxyethyl]-1-methyl-3-(4-hydroxyphenyl)propylamine and the corresponding S,R optical isomer. Separation of such diastereomers can be achieved by routine methods including chromatography. Alternatively, the mixture of such diastereomers can be utilized as such according to this invention since the S,R isomers are substantially devoid of biological activity.

A preferred group of compounds provided by this invention are those phenethanolamines having the above general formula in which one or both of the phenyl rings bear a hydroxyl group. When preparing such phenethanolamines which have one or more phenolic hydroxyl groups, it may be desirable to derivatize such groups so as to protect them during chemical reactions, thus avoiding any interference which might be caused by a free phenolic hydroxyl group. Phenolic hydroxyl groups can be protected with any of a number of commonly used hydroxyl protecting groups. The use of such groups is described in detail by E. Haslam in Protective Groups In Organic Chemistry, J.F.W. McOmie, Ed., Plenum Press, New York, N.Y. 1973, Chapter 3. Examples of commonly used protecting groups include ether forming groups such as benzyl, methyl, methoxymethyl, trimethylsilyl; ester forming groups such as acetate, benzoate, 2,2,2-trichloroacetate, phenylsulfonate, and related groups.

A typical example of the preparation of a phenethanolamine of this invention wherein one or both of $R_2$ and $R_3$ are hydroxy includes as a first step the preparation of a protected hydroxy phenyl propylamine derivative. For instance, a compound such as methyl 2-(3-hydroxyphenyl)ethyl ketone can be reacted with benzyl chloride under normal alkylation conditions to provide methyl 2-(3-benzyloxyphenyl)ethyl ketone. The ketone is reacted with ammonia to provide an imine, which then is reduced to afford the propylamine, namely 1-methyl-3-(3-benzyloxyphenyl)propylamine. The amine is resolved according to standard procedures to provide the optically active R isomer. The R-1-methyl-3-(3-benzyloxyphenyl)propylamine is next coupled to an optically active mandelic acid derivative such as R-2-(4-benzyloxyphenyl-2-hydroxy)acetic acid. The product of such coupling reaction is the corresponding amide, R,R-N-[2-(4-benzyloxyphenyl)-2-hydroxy-1-oxoethyl]-1-methyl-3-(3-benzyloxyphenyl)-propylamine. Reduction of the amide carbonyl group by reaction with diborane or a similar reducing agent affords a doubly protected phenethanolamine. The two benzyl protecting groups are readily removed by hydrogenation in the presence of Raney Nickel, thereby affording a compound of this invention, namely R,R-N-[2-(4-hydroxyphenyl)-2-hydroxyethyl]-1-methyl-3-(3-hydroxyphenyl)propylamine.

According to a further aspect of the invention there is provided a method of preparing a compound of formula (I) in which $R_2$ and/or $R_3$ is hydroxy which comprises deprotecting a compound of formula (V):

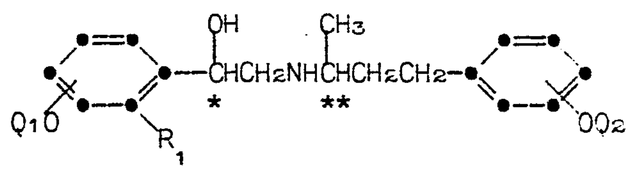

where $R_1$ is as previously defined and $Q_1$ and $Q_2$ are the same or different hydroxy protecting groups, or one of $Q_1$ and $Q_2$ is hydrogen. The nature of such protecting groups will be well-known to those skilled in the art. In the case of doubt, recourse may be made to the standard treatise <u>Protective Groups in Organic Chemistry</u> mentioned above. $Q_1$ and $Q_2$ are preferably benzyl groups capable of removable by standard hydrogenolysis conditions.

Since the compounds of this invention are phenethanolamine derivatives, they are basic in nature by virtue of the amino group. As such, the compounds are capable of forming salts with any of a number of inorganic and organic acids. An additional embodiment of this invention encompasses the pharmaceutically acceptable salts formed by reaction of a phenethanolamine of the above general formula with an acid. The term "pharmaceutically acceptable salt" as used herein refers to those phenethanolamine acid addition salts which can be utilized in a biological system without

imparting undesirable side effects attributable to the particular acid utilized to form the salt. While the identity of the particular acid used to form a pharmaceutically acceptable salt is not critical, the salt which is formed utilizing such acid must be pharmaceutically acceptable. Acids commonly utilized to form salts according to this invention include mineral acids such as hydrochloric, hydrobromic, phosphoric, sulfuric, perchloric, nitric, and related acids. Routinely used organic acids include acetic, butyric, citric, maleic, succinic, fumaric, lactic, methanesulfonic, p-toluenesulfonic, and similar organic acids.

Like most amine salts, the pharmaceutically acceptable salts comprehended by this invention are characteristically highly crystalline solids, and thus lend themselves to ready purification by recrystallization from common solvents such as ethanol, methanol, acetone, water, and the like. As will be described more fully hereinbelow, the phenethanolamine salts of the invention are often preferred as medicaments since they are easily formulated for convenient oral or parenteral administration. It will of course be recognized that a phenethanolamine acid addition salt can readily be converted to the free amine simply by reaction with a base such as aqueous sodium hydroxide, potassium hydroxide or sodium carbonate.

In an effort to further illustrate the various phenethanolamines comprehended by this invention, the following list of typical compounds is provided.

R,R-N-[2-(3-hydroxyphenyl)-2-hydroxyethyl]-1-methyl-3-(4-hydroxyphenyl)propylamine;

R,R-N-[2-(4-hydroxyphenyl)-2-hydroxyethyl]-1-methyl-3-(3-hydroxyphenyl)propylamine;

R,R-N-[2-(4-hydroxyphenyl)-2-hydroxyethyl]-1-methyl-3-(3-methoxycarbonylphenyl)propylamine;

R,R-N-[2-(2-fluorophenyl)-2-hydroxyethyl]-1-methyl-3-(4-hydroxyphenyl)propylamine;

R,R-N-[2-(2-fluorophenyl)-2-hydroxyethyl]-1-methyl-3-(4-methoxycarbonylphenyl)propylamine;

R,R-N-[2-(4-hydroxyphenyl)-2-hydroxyethyl]-1-methyl-3-(4-hydroxyphenyl)propylamine;

R,R-N-[2-(3-hydroxyphenyl)-2-hydroxyethyl]-1-methyl-3-(3-methoxycarbonylphenyl)propylamine;

R,R-N-[2-phenyl-2-hydroxyethyl]-1-methyl-3-(4-methylaminocarbonylphenyl)propylamine;

R,R-N-[2-(4-hydroxyphenyl)-2-hydroxyethyl]-1-methyl-3-(4-methoxycarbonylphenyl)propylamine;

R,R-N-[2-(3-hydroxyphenyl)-2-hydroxyethyl]-1-methyl-3-(4-fluorophenyl)propylamine;

R,R-N-[2-(2-fluorophenyl)-2-hydroxyethyl]-1-methyl-3-(4-aminocarbonylphenyl)propylamine;

R,R-N-[2-(2-fluorophenyl)2-hydroxyethyl]-1-methyl-3-(4-methylaminocarbonylphenyl)propylamine;

R,R-N-[2-(4-hydroxyphenyl)2-hydroxyethyl]-1-methyl-3-(4-hydroxyphenyl)propylaminium bromide;

R,R-N-[2-phenyl-2-hydroxyethyl]-1-methyl-3-(4-aminocarbonylpropyl)propylaminium acetate;

R,R-N-[2-(2-fluorophenyl)-2-hydroxyethyl]-1-methyl-3-(4-aminocarbonylphenyl)propylaminium chloride;

R,R-N-[2-(4-hydroxyphenyl)-2-hydroxyethyl]-1-methyl-3-(3-hydroxyphenyl)propylaminium oxolate;

R,R-N-[2-(3-hydroxyphenyl)-2-hydroxyethyl]-1-methyl-3-(4-methoxycarbonylphenyl)propylaminium phosphate;

R,R-N-[2-(3-hydroxyphenyl)-2-hydroxyethyl]-1-methyl-3-(4-methylaminocarbonylphenyl)propylaminium benzoate;

R,R-N-[2-(2-fluorophenyl)-2-hydroxyethyl]-1-methyl-3-phenylpropylaminium para-toluenesulfonate;

R,R-N-[2-(4-hydroxyphenyl)-2-hydroxyethyl]-1-methyl-3-(4-aminocarbonylphenyl)propylaminium phosphate;

R,R-N-[2-(4-hydroxyphenyl)-2-hydroxyethyl]-1-methyl-3-(4-hydroxyphenyl)propylaminium naphthalene sulfonate;

R,R-N-[2-(4-hydroxyphenyl)-2-hydroxyethyl]-1-methyl-3-(4-hydroxyphenyl)propylaminium isobutyrate;

R,R-N-[2-(3-hydroxyphenyl)-2-hydroxyethyl]-1-methyl-3-(4-aminocarbonylphenyl)propylaminium picrate;

R,R-N-(2-(2-fluorophenyl)-2-hydroxyethyl]-1-methyl-3-(4-methoxycarbonylphenyl)propylaminium bromide; and

R,R-N-(2-(3-hydroxyphenyl)-2-hydroxyethyl]-1-methyl-3-(4-fluorophenyl)propylaminium para-bromophenylsulfonate.

Additional aspects of this invention include a method for treating conditions of depressed cardiac contractility, a method for physically conditioning a biological system, and a pharmaceutical formulation comprising a phenethanolamine of the general formula (I) in combination with a suitable pharmaceutical carrier or diluent therefor. The compounds of this invention are uniquely active in effecting an increase in cardiac pumping force while not imparting the adverse conditions generally observed with the use of currently known cardiac drugs. In other words, the compounds of this invention are effective in increasing cardiac contractility while causing neither a substantial drop in blood pressure nor a substantial change in heart rate. The compounds of this invention are additionally unique in pharmacological properties due to their immediate onset, they are direct acting, they do not cause vasoconstriction, they cause little or no central nervous system effects, and they provide a low risk of arrhythmia.

Because the compounds comprehended by this invention have unique biological effects, it has been found that they can be used to physically condition a subject. The term "physically conditioning" as used herein is synonymous with increasing oxidative capacity of skeletal muscle. It now is widely recognized that physical training benefits people with occlusive peripheral or coronary artery disease. The result of such physical exercising is that skeletal muscle

adapts in such a way that it can make better use of available blood flow. From the physiologic standpoint, physical training causes an increase in the number or size, or both, of mitochondria, thereby enhancing the ability of skeletal muscle to utilize oxygen. This in turn permits the muscle to do more work on the same or lesser amount of blood flow. Therefore, a restriction of blood flow caused by a diseased artery supplying the skeletal muscle becomes less of a handicap. Moreover, since less blood flow is required by skeletal muscle, the demands on the heart are decreased so that the heart's need for blood flow is also reduced. Additionally, a restriction of blood flow caused by obstructive coronary artery disease in the heart becomes less of a handicap.

A further benefit occurring with physical training is a decrease in heart rate. Heart rate determines the number of times per minute that the heart develops blood pressure, and naturally a decrease in heart rate lowers the requirement for cardiac blood flow. Surprisingly, the compounds provided by this invention cause the same cardiovascular adaptations as does physical training, namely increased oxidative capacity of skeletal muscle, reduced cardiac blood flow requirements and a significant reduction in heart rate, both at rest and during physical activity such as walking and running. The compounds provided herein thus represent a significant advance in the art, since many of the people who need physical training the most are unable to exercise

because of physical handicaps, arthritis, or peripheral vascular disease or existing heart disease such that exercise may either precipitate or exacerbate symptoms of heart failure. By chronic treatment with a compound of this invention, the effects of physical conditioning can be realized.

According to the present method of treating heart failure, a dose effective for increasing cardiac contractility of a compound having the above formula is administered to a subject suffering from heart failure due to a condition of depressed cardiac contractility and inadequate ventricular function or inadequate pumping function or to a subject suspected of developing heart failure due to conditions of inadequate cardiac pumping function. The compounds can be formulated for convenient oral, topical, rectal or parenteral administration, and typically are administered in the form of a non-toxic pharmaceutically acceptable acid addition salt. The particular route of administration may vary according to the particular condition and subject to be treated. In cases of severe heart failure due to acutely depressed cardiac contractility, it may be desirable to administer a phenethanolamine of this invention intravenously until the pumping function is improved, at which time the patient can be maintained by intramuscular or oral administration. Additionally, the compounds of this invention can be formulated for administration via buccal seals, by sublingual lozenges,

by rectal suppositories, by metered aerosols and by dermal application. These latter named routes of administration are particularly preferred in the prophylactic treatment of inadequate ventricular function for which the compounds of this invention are well suited.

For intravenous administration according to the method of treatment of this invention, an optically active phenethanolamine of the above general formula is administered at the rate of about 0.01 to about 10 mcg./kg./min. until the contractile force of the heart muscle is restored. The infusion at such rate is continued until the subject shows signs of relief, at which time the rate of infusion can be decreased, for example to a rate of about 0.01 to about 10 mcg./kg./10 minutes, or as needed.

Maintenance therapy can be accomplished if desired by the intramuscular injection of from about 5 to about 90 mcg./kg. of body weight at intervals of from about one to four times per day, or as required by the severity of the cardiac condition being treated as well as the tolerance displayed by the patient. Maintenance therapy also is conveniently accomplished by oral administration of capsules containing sufficient quantity of a compound of this invention so that the effective dose is from about 5 to about 90 mcg./kg. of body weight. Since the acid addition salts of the phenethanolamine bases of the above formula are characteristically highly soluble in

water, it is preferred that oral administration is carried out utilizing formulations containing a phenethanolamine in the form of a pharmaceutically acceptable acid addition salt. For example, a particularly preferred method of treatment according to this invention comprises administering a dose effective for increasing the pumping force of a heart muscle of R,R-N-[2-(4-hydroxyphenyl)-2-hydroxyethyl]-1-methyl-3-(4-hydroxyphenyl)propylamine as the hydrochloride salt. Such compound is ideally administered orally, sublingually, rectally or dermally at a dose of from about 5 to about 150 mcg./kg. to a subject suffering from heart failure, or prophylactically to a subject suspected of developing heart failure due to conditions of inadequate heart pumping function.

As already pointed out, an additional embodiment of this invention is a method for physically conditioning a subject by administering an effective dose of a phenethanolamine of this invention. Such method accomplishes the physiological effects of physical exercise in subjects unable or unwilling to attain such conditioning by actual physical training. According to such method, a compound of this invention is administered to a subject at a dose of about 5 mcg./kg. to about 200 mcg./kg. Preferably, the compound is initially administered at a relatively low dose, for instance from about 5 to about 10 mcg./kg., at intervals of once or twice each day for about one or two weeks. The effective dose then is increased to

a dose of about 10 to about 20 mcg./kg. once or twice each day for about one or two weeks. The drug dose is then again increased at regular intervals until the maximum degree of physical conditioning is experienced without an adverse increase in heart rate. For example, such dose build up procedure can be carried out by administering effective amounts of a compound such as R,R-N-(2-phenyl-2-hydroxyethyl)-1-methyl-3-(4-aminocarbonylphenyl)propylaminium bromide. Such administration is preferably carried out orally at an initial dose of about 10 mcg./kg. Alternatively, such compound can be administered dermally in the form of a cream at an initial dose of about 1.0 to about 20 mg./kg. The dosage, by whichever route selected, is gradually increased until the desired degree of conditioning of the biological system is established. The oral administration of a compound such as R,R-N-(2-phenyl-2-hydroxyethyl)-1-methyl-3-(4-aminocarbonylphenyl)propylaminium bromide may reach, for instance, an upper oral limit of about 60 to about 90 mcg./kg. given from one to about four times each day. Such dosage will be continued as needed to maintain the desired effects of physical conditioning. Such maintenance therapy may be accomplished by the administration of the drug in the amount of about 60 to about 90 mcg./kg. from about one to four times per week as required.

The compounds of this invention can be formulated by any of a number of commonly used methods for convenient administration by routes such as orally, intravenously, intramuscularly, dermally, sublingually, rectally, via aerosols, buccal seals, and the like. Such pharmaceutical formulations are a further embodiment of this invention. A phenethanolamine of the invention, as the free base or acid addition salt, can be admixed with commonly used diluents and carriers, including starch powder, sucrose, dextrose, cellulose fiber, sorbitol, mannitol, propylene glycol, liquid paraffin, calcium silicate, silica, polyvinylpyrrolidone, cocoa butter, methyl cellulose, methyl hydroxybenzoate, ethyl lactate, sorbitan trioleate and related excipients and carriers. The formulations of this invention can additionally contain more than one active ingredient of this invention, as well as the pharmacologically less inotropicly active isomers such as the S,R-isomers. Such formulations generally will contain from about 0.1 to about 10 percent by weight of active ingredient. The formulated phenethanolamines can be encapsulated by an ingestible carrier in the form of a capsule, sachet, cachet, buccal seal, or the like, thus providing for convenient oral administration. Alternatively the formulations can be molded into tablets in unit dosage form. For intravenous administration, the formulated phenethanolamine is best dissolved in a suitable solvent such as isotonic saline or glucose or sterile water.

The phenethanolamines provided by this invention have been evaluated for their inotropic potency in anesthetized dogs and in conscious dogs with implanted cardiovascular transducers. The heart rate, arterial pressure, cardiac output, and the derivative of left ventricular pressure (which is the index of cardiac contractility), were measured. The compounds provided by this invention proved to be strongly inotropic, direct acting with immediate onset and adequate duration of action, and have displayed no alpha receptor activity. As hereinbefore pointed out, this invention comprehends optically active R,R-phenethanolamines of the above formula, since the R,S isomers are substantially devoid of inotropic activity. The R,S isomers are, however, effective in inducing the loss of adipose tissue in obese animals without imparting cardiac effects and are therefore useful as anti-obesity drugs. The unique and unexpected separation in pharmacological activity between the R,R-phenethanolamines of this invention and the R,S isomers is illustrated in Table I.

Table I

| Compound | Cardiac Contractile Potency mcg/kg $ED_{50}$ | Heart rate Change beats/min. at $ED_{50}$ | Blood Pressure Change mm/hg at $ED_{50}$ |
|---|---|---|---|
| R,R-N-[2-(4-hydroxyphenyl)-2-hydroxyethyl]-1-methyl-3-(4-hydroxyphenyl)propylamine | 0.32 | 15 | -5 |
| R,S-N-[2-(4-hydroxyphenyl)-2-hydroxyethyl]-1-methyl-3-(4-hydroxyphenyl)propylamine | 11 | 15 | -2 |
| R,R-N-(2-phenyl-2-hydroxyethyl)-1-methyl-3-(4-hydroxyphenyl)propylamine | 6 | 15 | -13 |
| R,S-N-[2-phenyl-2-hydroxyethyl)-1-methyl-3-(4-hydroxyphenyl)propylamine | 350 | 18 | -20 |
| R,R-N-(2-phenyl-2-hydroxyethyl)-1-methyl-3-(4-aminocarbonylphenyl)propylamine | 7 | 15 | +3 |
| R,S-N-(2-phenyl-2-hydroxyethyl)-1-methyl-3-(4-aminocarbonylphenyl)propylamine | 1984 ($ED_{max}$)[1] | 24 ($ED_{max}$) | -30 ($ED_{max}$) |

[1] $ED_{max}$ indicates that a 50% increase in contractile force could not be achieved. The maximal increase achieved with the indicated dose was approximately 45%.

The compounds provided herein also have been evaluated as physical conditioning agents. In a typical experiment, a group of ten dogs were implanted with miniature pressure transducers in their left ventricle so that measurements of heart rate, systolic pressure and the derivative of left ventricular pressure (the index of cardiac contractility) could be made. The dogs were trained to walk on a treadmill. Five of the dogs were randomly selected to receive a compound of this invention, for instance R,R-N-[2-(4-hydroxyphenyl)-2-hydroxyethyl]-1-methyl-3-(4-hydroxyphenyl)propylaminium chloride. The test animals received saline filled capsules during the first week of the test. During the second and third weeks, the dogs received the test compound orally at a dose of 10 mcg./kg. twice each day. The dosage was increased to 40 mcg./kg. twice each day for the fourth week, and increased to 60 mcg./kg. twice each day through weeks five to eleven. The other five dogs received saline filled capsules throughout the study. Once each week, the animals' response to exercise on the treadmill was evaluated. Resting data were obtained by permitting the dogs to lie on the treadmill before turning it on. The treadmill then was run at a speed of 3.5 kilometers/hr., with the dogs walking at this speed with no elevation for 5 min., and then for 5 min. at a 20% incline. During the eleventh week of such test, the dogs were made to walk on a 40% incline, thus providing sufficient stress to

increase plasma lactic acid and catecholamines. Blood samples prior to each exercise and within two minutes of completing the exercise were taken from the jugular vein.

After several weeks, the dogs were anesthetized and measurements of ventricular function and skeletal muscle blood flow and enzymes were made. The results demonstrated that, for the placebo treated dogs, no significant change in heart rate occurred during the course of the experiment, whereas the heart rate both while resting and while exercising was significantly reduced in the dogs conditioned with a compound of this invention. Moreover, skeletal muscle blood flow was significantly reduced, because the muscle's capacity to utilize oxygen was significantly enhanced as demonstrated by a significantly enhanced activity of succinic dehydrogenase. Further evidence of the compound's conditioning effect was obtained by measuring circulating lactic acid and norepinephrine. During exercise on the treadmill, lactic acid and norepinephrine blood concentrations were significantly lower in the dogs conditioned with a compound of this invention than in those cases where the dogs received only placebo. These results are presented in Table II.

Because of the ability of the compounds of this invention to effect the same result in a biological system which is accomplished by physical exercise, the compounds can be used to physically condition an animal unable or unwilling to physically

exercise, and can be used to further physically condition an animal which undergoes physical exercise. Ideally, the pure R,R-isomers of this invention are administered at a dose of about 5 to about 90 mcg./kg. from 1 to 4 times per day to a human desirous of becoming physically conditioned. It may be economically desirable to utilize all four optical isomers as a mixture in the conditioning of animals such as racing dogs and horses.

TABLE II

| | Blood Lactic Acid (mg.%) | | Plasma Norepinephrine (picograms/ml.) | |
|---|---|---|---|---|
| | at rest | during exercise | at rest | during exercise |
| | p<0.01 | | p<0.05 | |
| Dogs dosed with | | | | |
| R,R-N-[2-(4-hydroxyphenyl)-2-hydroxyethyl]-1-methyl-3-(4-hydroxyphenyl)propylaminium chloride | 5.2 | 6.3 | 235 | 200 |
| Placebo treated dogs | 6 | 10 | 270 | 380 |

TABLE II (continued)

| | Skeletal Muscle Blood Flow (ml./min./100 g.) | Succinic Dehydrogenase Activity (μM/mgP/min.) | Heart Rate (beats/min.) at rest | Heart Rate (beats/min.) at 20° walk |
|---|---|---|---|---|
| | $p<0.01$ | $p<0.01$ | $p<0.01$ | |
| R,R-N-[2-(4-hydroxyphenyl)-2-hydroxyethyl]-1-methyl-3-(4-hydroxyphenyl)propylaminium chloride | 2.5 | 3.3 | 55 | 150 |
| Placebo treated dogs | 3.5 | 2 | 85 | 170 |

The following detailed Preparations and Examples are provided by way of illustration of various specific aspects of the invention.

Preparation 1

Preparation of dl-4-(benzyloxy)mandelic acid

A solution of 5.0 g. of dl-4-hydroxy mandelic acid, 11.0 g. of benzyl chloride and 10.0 g. of potassium carbonate in 50 ml. of methanol was heated to reflux and stirred for seventy-two hours. The reaction mixture was cooled to room temperature and diluted with 50 ml. of water. The aqueous solution was washed twice with 50 ml. portions of benzene, and then was acidified with concentrated hydrochloric acid. The aqueous acid solution was extracted three times with 50 ml. portions of ethyl acetate. The organic extracts were combined, washed with water and with saturated sodium chloride solution and dried. Removal of the solvent by evaporation under reduced pressure provided 5.7 g. of a solid which was then recrystallized from 300 ml. of toluene to afford 5.33 g. of dl-4-(benzyloxy)mandelic acid. M.P. 153-155°C.

Analysis calc. for $C_{15}H_{14}O_4$

Theory: C, 69.76; H, 5.46.

Found : C, 69.96; H, 5.33.

## Preparation 2

Resolution of dl-4-(benzyloxy)mandelic acid to provide R(-)-(4-benzyloxy)mandelic acid

To a stirred solution of 185.6 g. of dl-4-benzyloxy)mandelic acid in 2500 ml. of ethyl acetate at 80°C. was added in one portion 43.6 g. of R(+)-α-methylbenzylamine. The reaction mixture was cooled to room temperature, and the precipitated solid which had formed was collected by filtration and washed with fresh ethyl acetate. The solid was recrystallized twice from a solution of ninety percent ethanol in water to provide 91.4 g. of the R(+)-α-methylbenzylamine salt of R(-)-4-(benzyloxy)mandelic acid. M.P. 208.5-209.5°C. $[\alpha]_D$ -38.6°, $[\alpha]_{365}$ -155.3° (MeOH)

Analysis calc. for $C_{23}H_{25}NO_4$

Theory: C, 72.80; H, 6.64; N, 3.69.

Found : C, 72.95; H, 6.83; N, 3.95.

To a stirred suspension of 91.4 g. of the above-named salt in 2000 ml. of ethyl acetate was added 150 ml. of ten percent aqueous hydrochloric acid solution. The aqueous acid solution was separated, and the organic layer washed with water and dried. Removal of the solvent by evaporation under reduced pressure afforded 54.5 g. of R(-)-4-(benzyloxy)mandelic acid, ie. R(-)-2-(4-benzyloxyphenyl)-2-hydroxyacetic acid. M.P. 155-161°C. $[\alpha]_D$ -102.2°; $[\alpha]_{365}$ -410.6° (MeOH)

Analysis calc. for $C_{15}H_{14}O_4$

Theory: C, 69.76; H, 5.46.

Found : C, 69.67; H, 5.41.

## Preparation 3

Preparation of dl-1-methyl-3-(4-benzyloxyphenyl)-propylamine

A solution of 40.0 g. of methyl 2-(4-benzyloxyphenyl)ethyl ketone (prepared by benzylation of the corresponding 4-hydroxyphenyl derivative) and 160 ml. of anhydrous ammonia in 300 ml. of ethanol was heated at 75°C. and stirred for two hours. After cooling the reaction mixture to room temperature, 4.0 g. of Raney nickel was added in one portion, and the reaction mixture then was stirred at 25°C. for twelve hours under a hydrogen atmosphere at 300 psi. The reaction mixture next was filtered and the filtrate was concentrated by evaporation of the solvent under reduced pressure to provide an oil. The oil was dissolved in 120 ml. of 3N hydrochloric acid, from which the product as a hydrochloride salt precipitated. The salt so formed was collected by filtration and recrystallized from methanol and toluene to provide 36.2 g. of dl-1-methyl-3-(4-benzyloxyphenyl)propylaminium chloride. M.P. 195-197.5°C.

## Preparation 4

Preparation of R-1-Methyl-3-(4-hydroxyphenyl)propylamine

A solution of 1.83 g. of R-1-methyl-3-(4-benzyloxyphenyl)propylaminium chloride, m.p. 176-178°C. obtained from the free base by treatment with hydrochloric acid, in ethanol was hydrogenated with 0.5 g. of five percent of palladium-on-carbon.

After removal of the catalyst and the solvent, there was obtained 1.21 g. of solid product. Recrystallization from acetonitrile and ethanol afforded 0.82 g. of R-1-methyl-3-(4-hydroxyphenyl)propylaminium chloride, m.p. 204-206.5°C. $[\alpha]_D$ +8.1° (MeOH).

Analysis calc. for $C_{10}H_{16}ClNO$

Theory: C, 59.55; H, 8.00; N, 6.94.

Found : C, 59.56; H, 7.88; N, 7.13.

## Preparation 5

Resolution of dl-1-methyl-3-(4-benzyloxyphenyl)-propylamine to provide R-(-)-1-methyl-3-(4-benzyloxyphenyl)propylamine

A solution of 629.3 g. of dl-1-methyl-3-(4-benzyloxyphenyl)propylaminium chloride was converted to the free amine by reaction with 95 g. of sodium hydroxide in 400 ml. of water. The free amine was then dissolved in 2000 ml. of methylene chloride and added to a solution of 328 g. of D-(-)-mandelic acid in 1000 ml. of methanol. The mandelic acid salt of the free amine precipitated out of solution and was recrystallized three times from methanol to provide 322 g. of the R-mandelic acid salt of R-1-methyl-3-(4-benzyloxyphenyl)propylamine. M.P. 166-167°C. $[\alpha]_D$ -30°, $[\alpha]_{365}$ -119° (MeOH).

The salt so formed was converted to R-1-methyl-3-(4-benzyloxyphenyl)propylamine as the free base by reaction with aqueous sodium hydroxide.

## Preparation 6

R-1-Methyl-3-(4-aminocarbonylphenyl)propylaminium chloride

The oil from 280 g. of a 50% dispersion of sodium hydride in mineral oil was removed by several washings with hexane. The hexane was replaced with

1.5 l. of dimethylformamide, and the mixture was stirred and cooled in an ice-bath while 950 g. of ethyl acetoacetate was slowly added. After the sodium hydride had reacted a solution of 314 g. of α-bromo-p-toluic acid in 500 ml. of dimethylformamide was added. The ice bath was removed, and the mixture was allowed to stir at 25°C. for eighteen hours, poured into water acidified with 3N hydrochloric acid and extracted with ethyl acetate. The combined organic layers were washed with water and brine and dried over anhydrous sodium sulfate. The solvent and excess ethyl acetoacetate were removed at reduced pressure to afford 500 g. of oil.

The crude oil was combined with 1.2 l. of glacial acetic acid, 140 ml. of concentrated hydrochloric acid and 750 ml. of water and heated at reflux for three and one half hours. The solvents were removed under reduced pressure and the residue was dissolved in a mixture of ether and xylene and washed with water. The solid obtained after removal of the solvents was recrystallized twice from a mixture of ethanol and water to give 111 g. of 4-(4-carboxyphenyl)-2-butanone, m.p. 120.5-123°C.

Analysis calc. for $C_{11}H_{12}O_3$

Theory: C, 68.74; H, 6.29.

Found : C, 68.99; H, 6.10.

A mixture of 9.50 g. of 4-(4-carboxyphenyl)-2-butanone, 100 ml. of benzene, 15.7 g. of oxalylchloride and one drop of dimethylformamide was stirred and warmed to 50°C. for two hours. The reaction mixture was then allowed to stir for eighteen hours at 25°C. and concentrated under reduced pressure. The residue was dissolved in 50 ml. of dioxane and, with stirring, was added slowly to 300 ml. of cold (0°C.) concentrated ammonium hydroxide. The mixture was stirred for seventy-five minutes and diluted to 1 l. with water. The solid was removed by filtration, washed with water, dried and recrystallized from a mixture of methanol and ether to provide 4.1 g. of 4-(4-aminocarbonylphenyl)-2-butanone, m.p. 144-146°C. An analytical sample, m.p. 149-150°C., was obtained by recrystallizing twice from a mixture of methanol and ether.

Analysis calc. for $C_{11}H_{13}NO_2$

Theory: C, 69.09; H, 6.85; N, 7.32.

Found : C, 68.98; H, 7.10; N, 6.99.

A mixture of 61.8 g. of 4-aminocarbonylphenyl-2-butanone, 39.2 g. of R-(+)-α-methylbenzylamine 0.5 g. of p-toluenesulfonic acid and 600 ml. toluene were stirred and heated to reflux. The 5 ml. of water that formed during the reaction was removed azeotropically.

The solvent was removed under reduced pressure and the residue was dissolved in 500 ml. of

methanol and hydrogenated in the presence of 25 g. of Raney nickel catalyst at ambient temperature.

After removal of the catalyst by filtration and the solvent by evaporation under reduced pressure, the residue was dissolved in methanol and treated with an excess of hydrochloric acid method. Recrystallization of the solid from methanol afforded 42.1 g. Two crystallizations from a mixture of methanol and acetonitrile gave 34 g. of R,R-N-(α-methylbenzyl)-1-methyl-3-(4-aminocarbonylphenyl)-propylaminium chloride, m.p. 256-257°C., $[\alpha]_D$ +79.2° (MeOH).

A solution of 33 g. of the salt in 860 ml. of ethanol containing 5 g. of 5 percent palladium-on-carbon was hydrogenated for six hours at 50°C. The catalyst was removed by filtration and the solvent was evaporated under reduced pressure to provide the product. Several recrystallization from ethanol and ethyl acetate gave 8.2 g. of R-1-methyl-3-(4-aminocarbonylphenyl)propylaminium chloride, m.p. 241-242° $[\alpha]_D$ +7.9°.

Analysis calc. for $C_{11}H_{17}ClN_2O$

Theory: C, 57.76; H, 7.49; N, 12.25.

Found : C, 58.01; H, 7.32; N, 12.13.

Example 1

R,R-N-[2-(4-Benzyloxyphenyl)-2-hydroxy-1-oxoethyl]-1-methyl-3-(4-benzyloxyphenyl)propylamine

A solution of 93.9 g. of R-1-methyl-3-(4-benzyloxyphenyl)propylamine in 500 ml. of N,N-dimethylformamide containing 63.0 g. of 1-hydroxybenzotriazole and 104.6 g. of R-2-(4-benzyloxyphenyl)-2-hydroxyacetic acid was cooled to 0°C. and stirred while a solution of 83.6 g. of N,N'-dicyclohexylcarbodiimide in 300 ml. of dimethylformamide was added dropwise over one hour. The reaction mixture was stirred for twelve hours at 3°C. and then was diluted with 10 ml. of water, stirred for an additional hour, and then cooled to -30°C. in an ice-acetone bath. The reaction mixture was filtered to remove dicyclohexylurea, and then the filtrate was concentrated by evaporation of the solvent under reduced pressure. The concentrated solution was diluted with ethyl acetate and washed with saturated

aqueous sodium carbonate solution, with water, with 300 ml. of 1N hydrochloric acid, and again with water. The organic layer was dried and the solvent was removed by evaporation under reduced pressure to provide the product as a white solid. The solid was recrystallized once from acetonitrile and again from methanol to provide 159.7 g. of R,R-N-[2-(4-benzyloxyphenyl)-2-hydroxy-1-oxoethyl]-1-methyl-3-(4-benzyloxyphenyl)propylamine. M.P. 145-148°C. $[\alpha]_D$ -15.9°, $[\alpha]_{365}$ -50.1° (MeOH).

Analysis calc for $C_{32}H_{33}NO_4$

Theory: C, 77.55; H, 6.71; N, 2.83.

Found : C, 77.04; H, 6.84; N, 2.53.

## Example 2

R,R-N-[2-(4-Benzyloxyphenyl)-2-hydroxyethyl]-1-methyl-3-(4-benzyloxyphenyl)propylamine

To a stirred solution of 10.0 g. of R,R-N-[2-(4-benzyloxyphenyl)-2-hydroxy-1-oxoethyl]-1-methyl-3-(4-benzyloxyphenyl)propylamine in 500 ml. of freshly distilled tetrahydrofuran under a nitrogen gas atmosphere was added dropwise over thirty minutes 41 ml. of 2 molar borane-dimethyl sulfide complex in tetrahydrofuran. The reaction mixture was stirred at 25°C. for twenty hours, and then was heated to reflux and stirred for an additional three hours. After cooling the reaction mixture to 25°C. and stirring for another eighteen hours, the excess borane was decomposed by the slow portion-wise addition of 400 ml. of methanol. The solvent was then removed from the reaction mixture by evaporation under reduced pressure

to provide the product as an oil. The oil so formed was dissolved in 250 ml. of hot methanol, and after concentrating the volume to 125 ml., the product began crystallizing out of solution. The crystalline product was collected by filtration and recrystallized twice from methanol to provide 6.65 g. of R,R-N-[2-(4-benzyloxyphenyl)-2-hydroxyethyl]-1-methyl-3-(4-benzyloxyphenyl)propylamine. M.P. 119-123.5°C.

The amine so formed was dissolved in methanol and added to a solution of ethereal hydrogen chloride, thereby providing 6.49 g. of R,R-N-[2-(4-benzyloxyphenyl)-2-hydroxyethyl]-1-methyl-3-(4-benzyloxyphenyl)-propylaminium chloride. M.P. 214.5-216°C. $[\alpha]_D$ -13.4°, $[\alpha]_{365}$ -30.2° (MeOH).

Analysis calc. for $C_{32}H_{36}NO_3Cl$

Theory: C, 74.19; H, 7.00; N, 2.70; Cl, 6.84.

Found : C, 74.20; H, 6.98; N, 2.65; Cl, 6.63.

## Example 3

R,R-N-[2-(4-Hydroxyphenyl)-2-hydroxyethyl]-1-methyl-3-(4-hydroxyphenyl)propylaminium chloride

A mixture of 51.6 g. of R,R-N-[2-(4-benzyloxyphenyl)-2-hydroxyethyl]-1-methyl-3-(4-benzyloxyphenyl)propylaminium chloride and 5.0 g. of Raney nickel in 2 liters of ethanol and 2 liters of ethyl acetate was stirred at 25°C. for four and one-half hours under a hydrogen atmosphere of 60 psi. The reaction mixture was then filtered to remove the residual Raney nickel, and the filtrate was concentrated to an oil by evaporation of the solvent under

reduced pressure, and the oil was crystallized from fresh ethanol and diethyl ether to provide 29.8 g. of R,R-N-[2-(4-hydroxyphenyl)-2-hydroxyethyl]-1-methyl-3-(4-hydroxyphenyl)propylaminium chloride. M.P. 176-176.5°C. (dec.) $[\alpha]_D$ -22.7°, $[\alpha]_{365}$ -71.2° (3.7 mg/ml MeOH).

Analysis calc. for $C_{18}H_{24}NO_3Cl$

Theory: C, 63.99; H, 7.16; N, 4.15.

Found: C, 63.70; H, 7.26; N, 4.32.

Example 4

R,R-N-(2-Phenyl-2-hydroxyethyl)-1-methyl-3-(4-methoxycarbonylphenyl)propylamine

R-Mandelic acid (5.58 g.) was coupled to 7.6 g. of R-1-methyl-3-(4-methoxycarbonylphenyl)-propylamine by reaction in 100 ml. of dimethylformamide in the presence of 5.2 g. of 1-hydroxybenzotriazole and 7.58 g. of N,N'-dicyclohexylcarbodiimide. The R,R-N-(2-phenyl-2-hydroxy-1-oxoethyl)-1-methyl-3-(4-methoxycarbonylphenyl)propylamine (9.98 g.) which was thus formed was dissolved in 200 ml. of dry tetrahydrofuran. The reaction mixture was stirred at 25°C. while a solution of 150 ml. of 1.02 normal diborane in tetrahydrofuran was added portionwise over thirty minutes. After complete addition of the diborane solution, the reaction mixture was stirred at 25°C. for twenty-five hours. The excess diborane was decomposed by adding 50 ml. of methanol to the stirred reaction mixture, and then the reaction mixture was further diluted by the addition of 100 ml. of diethyl

ether saturated with hydrogen chloride. The solvent was next removed by evaporation under reduced pressure to provide an oil which was then dissolved in 100 ml. of methanol. The methanolic solution was heated to about 65°C. and stirred for twenty minutes, after which time the remaining methanol was removed by evaporation. The residue was dissolved in methanol containing a dilute solution of diethyl ether and hydrogen chloride, out of which solution a crystalline product precipitated. The product was identified as R,R-N-(2-phenyl-2-hydroxyethyl)-1-methyl-3-(4-methoxycarbonylphenyl)propylaminium chloride.

Analysis calc. for $C_{20}H_{26}NO_3Cl$

Theory: C, 66.02; H, 7.20; N, 3.85.

Found: C, 66.25; H, 7.16; N, 4.14.

The amine salt thus formed was dissolved in 800 ml. of ethyl acetate and the solution was washed with 200 ml. of aqueous sodium carbonate, with water and then was dried. Removal of the solvent by evaporation under reduced pressure provided 2.80 g. of R,R-N-(2-phenyl-2-hydroxyethyl)-1-methyl-3-(4-methoxycarbonylphenyl)propylamine. M.P. 106-110°C. $[\alpha]_D$ -6.8°, $[\alpha]_{365}$ -6.1°, (MeOH).

Analysis calc. for $C_{20}H_{25}NO_3$

Theory: C, 73.37; H, 7.70; N, 4.28.

Found: C, 73.03; H, 7.77; N, 3.91.

## Example 5

R,R-N-(2-Phenyl-2-hydroxyethyl)-1-methyl-3-phenylpropylaminium chloride

A solution of 4.25 g. of lithium aluminum hydride in 500 ml. of diethyl ether was added dropwise over fifteen minutes to a stirred solution of 12.92 g. of R,R-N-(2-phenyl-2-hydroxy-1-oxoethyl)-1-methyl-3-phenylpropylamine in 60 ml. of diethyl ether. The reaction mixture then was heated to reflux and stirred for six hours. After cooling the reaction mixture to 25°C., it was stirred for an additional twelve hours, and then again heated to reflux for another hour. The mixture then was cooled carefully treated with 100 ml. of water, 50 ml. of 5 N sodium hydroxide, and again with 100 ml. of water. The organic layer was separated and dried and the solvent was removed by evaporation under reduced pressure to provide 11.6 g. of the product as a solid. The solid thus formed was dissolved in diethyl ether and acetonitrile, and the solution was diluted with an ethereal solution of hydrogen chloride. The crystalline precipitate which formed was collected by filtration and dried to provide R,R-N-(2-phenyl-2-hydroxyethyl)-1-methyl-3-phenylpropylaminium chloride. M.P. 165.5-167.5°C. $[\alpha]_D$ -14.84° $[\alpha]_{365}$ -58.13° ($H_2O$)

Analysis calc. for $C_{18}H_{24}NOCl$

Theory: C, 70.69; H, 7.91; N, 4.58; Cl, 11.59.

Found: C, 70.47; H, 7.73; N, 4.34; Cl, 11.71.

## Example 6

R,R-N-(2-Phenyl-2-hydroxyethyl)-1-methyl-3-(4-hydroxyphenyl)propylaminium chloride

R-1-Methyl-3-(4-benzyloxyphenyl)propylamine (10.0 g.) was acylated by reaction with 6.37 g. of R-2-phenyl-2-dichloroacetoxyacetyl chloride in dichloromethane in the presence of 5.0 g. of sodium bicarbonate to provide, after purification by chromatography, 7.5 g. of R,R-N-(2-phenyl-2-dichloroacetoxy-1-oxoethyl)-1-methyl-3-(4-benzyloxyphenyl)propylamine. M.P. 131-134°C.

The amide thus formed was further purified by crystallization from 500 ml. of diethyl ether and 100 ml. of dichloromethane, and then was reacted with 50 ml. of a 1 molar solution of diborane in tetrahydrofuran for eighteen hours at reflux temperature. After cooling the reaction mixture to room temperature and decomposing excess diborane by the addition of 4 ml. of water, the reaction mixture was diluted with 30 ml. of 3 N hydrochloric acid, and again heated to reflux and stirred for one hour. The reaction mixture next was cooled to 40°C. and made alkaline by the addition of 50 ml. of 5 N sodium hydroxide, then diluted with 50 ml. of ethyl acetate. The organic layer was separated, washed with water, dried, and the solvent was removed by evaporation under reduced pressure to provide the product as a solid. The solid so formed was recrystallized from 70 ml. of ethanol and then reacted with hydrogen chloride in diethyl ether to afford 5.72 g. of R,R-N-(2-phenyl-2-hydroxyethyl)-1-methyl-3-(4-benzyloxyphenyl)propylaminium chloride. M.P. 188.5-191°C.

Analysis calc. for $C_{25}H_{30}NO_2Cl$

Theory: C, 72.89; H, 7.34; N, 3.40; Cl, 8.61

Found: C, 73.12; H, 7.43; N, 3.57; Cl, 8.84.

A solution of 5.0 g. of R,R-N-(2-phenyl-2-hydroxyethyl)-1-methyl-3-(4-benzyloxyphenyl)propylaminium chloride in 100 ml. of methanol containing 500 mg. of five percent palladium suspended on carbon was hydrogenated for three hours at room temperature under a hydrogen atmosphere of 55 psi. The reaction mixture then was filtered and the filtrate was concentrated to dryness by evaporation of the solvent under reduced pressure. The residual product was crystallized from ethanol and acetone to provide 3.08 g. of R,R-N-(2-phenyl-2-hydroxyethyl)-1-methyl-3-(4-hydroxyphenyl)propylaminium chloride. M.P 177.5-178.5°C. $[\alpha]_D^{27°C.}$ -28.8°, $[\alpha]_{365}^{27°C}$ -67.7° (5.980 mg/5 ml MeOH).

Analysis calc. for $C_{18}H_{24}NO_2Cl$

Theory: C, 67.17; H, 7.52; N, 4.35; Cl, 11.02.

Found: C, 66.91; H, 7.26; N, 4.35; Cl, 11.65.

Example 7

R,R-N-(2-Phenyl-2-hydroxyethyl)-1-methyl-3-(3-hydroxyphenyl)propylamine

A solution of 5.56 g. of R-1-methyl-3-(3-hydroxyphenyl)propylamine in 80 ml. of tetrahydrofuran was added to a stirred solution of 6.0 g. of the cyclic carbonate anhydride of R-mandelic acid. The

reaction mixture was stirred at 25°C. for twenty-three hours, after which time the solvent was evaporated therefrom to provide an oily residue. The residue was dissolved in ethyl acetate and washed four times with 30 ml. portions of 3 N hydrochloric acid, and then with water. The solution was dried and the solvent evaporated therefrom to provide 10.5 g. of R,R-N-(2-phenyl-2-hydroxy-1-oxoethyl)-1-methyl-3-(3-hydroxyphenyl)propylamine.

A solution of 9.5 g. of the amide so formed dissolved in 500 ml. of hot benzene containing 50 ml. of triethylamine was stirred while 11 ml. of trimethylsilyl chloride was added portionwise over five minutes. The reaction mixture then was heated at reflux and stirred for four hours. The solution next was cooled and filtered and the solvent was removed from the filtrate by evaporation, thus leaving a solid residue which next was dissolved in 30 ml. of tetrahydrofuran. The mixture was stirred at room temperature while a solution of 2.0 g. of lithium aluminum hydride in 50 ml. of tetrahydrofuran was added dropwise over ten minutes. Following complete addition of the reducing agent, the reaction mixture was heated to reflux and stirred for seventy-two hours. The reaction mixture then was cooled to room temperature and diluted with 300 ml. of diethyl ether containing 5 ml. of water. The reaction mixture next was filtered through Celite, and then the solvent was removed by evaporation and the residue was re-dissolved in benzene. The benzene solution was added to 150 ml. of

1 N hydrochloric acid solution, and the mixture was stirred at 25°C. for one hour. The organic layer then was separated and the aqueous acid layer was made basic by the addition of solid sodium carbonate. The aqueous alkaline solution was extracted several times with fresh benzene. The extracts were combined, washed with water and dried. Removal of the solvent by evaporation under reduced pressure provided the product as an oil. The oil was dissolved in diethyl ether and the solution was made acidic by the addition of hydrogen chloride. The crystalline product which formed was collected by filtration and dried to provide 2.053 g. of R,R-N-(2-phenyl-2-hydroxyethyl)-1-methyl-3-(3-hydroxyphenyl)propylaminium chloride. M.P. 163.5-165.5°C. $[\alpha]_D$ -17.7°, $[\alpha]_{365}$ -52.3° (9.65 mg/5 ml MeOH)

Analysis calc. for $C_{18}H_{24}NO_2Cl$

Theory: C, 67.17; H, 7.52; N, 4.35; Cl, 11.02.

Found: C, 67.35; H, 7.36; N, 4.42; Cl, 11.22.

## Example 8

R,R-N-[2-(2-fluorophenyl)-2-hydroxyethyl]-1-methyl-3-(4-hydroxyphenyl)propylaminium chloride

R-Ortho-fluoromandelic acid (10.20 g.) was coupled to 15.30 g. of R-1-methyl-3-(4-benzyloxyphenyl)propylamine by reaction in dimethylformamide in the presence of 8.10 g. of 1-hydroxybenzotriazole and 12.36 of N,N'-dicyclohexylcarbodiimide to provide, after isolation and purification, 24.6 g. of R,R-N-[2-(2-fluorophenyl)-2-hydroxy-1-oxoethyl]-1-methyl-3-(4-benzyloxyphenyl)propylamine.

The amide thus formed was reduced by reaction with 16.1 g. of lithium aluminum hydride in 800 ml. of diethyl ether according to the procedure set forth in Example 11 above. The excess reducing agent was decomposed by addition to the reaction mixture of 17 ml. of water, 13 ml. of twenty percent aqueous sodium hydroxide, and an additional 59 ml. of water. The reaction mixture was filtered and the filtrate was concentrated in volume by evaporation of the solvent under reduced pressure. The residue thus formed was dissolved in diethyl ether and ethanol and added to a solution of hydrogen chloride in diethyl ether. The solid which precipitated was collected by filtration, dried and recrystallized from ethanol and diethyl ether to afford R,R-N-[2-(2-fluorophenyl)-2-hydroxyethyl]-1-methyl-3-(4-benzyloxyphenyl)propylaminium chloride. M.P. 176-177°C.

Analysis calc. for $C_{25}H_{29}NO_2FCl$

Theory: C, 69.84; H, 6.80; N, 3.26; F, 4.42; Cl, 8.25.

Found: C, 70.04; H, 6.84; N, 3.44; F, 4.30; Cl, 8.55.

A solution of 9.8 g. of the amine hydrochloride salt thus produced dissolved in 140 ml. of ethanol containing 2.0 g. of five percent palladium on carbon was hydrogenated for five and one-half hours at 25°C. under a hydrogen atmosphere of 50 psi. The reaction mixture then was filtered and the solvent was removed from the filtrate by evaporation under reduced

pressure to provide 7.8 g. of the product as a solid hydrochloride salt. The solid salt was recrystallized from ethanol and diethyl ether and from ethyl acetate and ethanol, and then was converted to the free amine by reaction with sodium carbonate. The free amine was dissolved in fresh diethyl ether, and the ethereal solution was diluted with a solution of hydrogen chloride in diethyl ether. The crystalline solid which precipitated was collected by filtration, dried and recrystallized from fresh ethanol and diethyl ether, thus providing R,R-N-[2-(2-fluorophenyl)-2-hydroxyethyl]-1-methyl-3-(4-hydroxyphenyl)propylaminium chloride. M.P. 180-183°C. $[\alpha]_D$ -20.1°, $[\alpha]_{365}$ -60.6° (149.132 mg/5 ml MeOH).

Analysis calc. for $C_{18}H_{23}NO_2FCl$

Theory: C, 63.62; H, 6.82; N, 4.12; F, 5.59; Cl, 10.43.

Found: C, 63.91; H, 7.12; N, 4.35; F, 5.36; Cl, 10.63.

## Example 9

R,R-N-(2-Phenyl-2-hydroxyethyl)-1-methyl-3-(4-aminocarbonylphenyl)propylaminium chloride

A solution of 2.62 g. of R,R-N-(2-phenyl-2-hydroxyethyl)-1-methyl-3-(4-methoxycarbonylphenyl)-propylamine (prepared as described in Example 4) in 100 ml. of ethanol containing 40 ml. of anhydrous hydrazine was heated at reflux for six and one-half hours, and then permitted to set at room temperature

for twelve hours. The solvent then was removed from the reaction mixture by evaporation under reduced pressure to provide a solid residue which was crystallized from 10 ml. of 2-propanol and n-hexane. The crystalline product was collected by filtration and dried to afford 2.11 g. of R,R-N-(2-phenyl-2-hydroxyethyl)-1-methyl-3-(4-hydrazinocarbonylphenyl)propylamine. M.P. 105-108°C. $[\alpha]_D$ -7.9°, $[\alpha]_{365}$ -10.1° (MeOH).

Analysis calc. for $C_{19}H_{25}N_3O_2$

Theory: C, 69.70; H, 7.70; N, 12.83.

Found: C, 69.59; H, 7.57; N, 12.56.

A solution of 1.9 g. of the hydrazine derivative thus prepared dissolved in 225 ml. of methanol containing 1.3 g. of Raney nickel was reacted with hydrogen in a Brown hydrogenator. The reaction continued for twelve hours at room temperature, after which time an additional 2 g. of Raney nickel and 3 g. of prehydrogenated Raney nickel was added to the reaction mixture and the reaction was continued for another three days. When none of the starting material remained in the reaction mixture, as demonstrated by thin layer chromatographic analysis, the reaction mixture was filtered and the solvent was removed from the filtrate by evaporation under reduced pressure. The residue thus produced was dissolved in 200 ml. of water and 30 ml. of 3 N hydrochloric acid. The aqueous acid solution was washed with ethyl acetate, and then made basic by the addition of ammonium

hydroxide. The product, as the free amine, was extracted from the aqueous alkaline solution into fresh ethyl acetate. The organic layer was separated, washed with water and dried. Evaporation of the solvent provided 1.3 g. of the desired product as a free amine. The product was dissolved in methanol and converted to a hydrochloride salt by the addition of an ethereal solution of hydrogen chloride. The solid which precipitated out of solution was collected by filtration and recrystallized from 25 ml. of acetonitrile and 25 ml. of methanol to provide 1.22 g. of R,R-N-(2-phenyl-2-hydroxyethyl)-1-methyl-3-(4-aminocarbonyl-phenyl)propylaminium chloride. M.P. 244-246°C. $[\alpha]_D$ -12°, $[\alpha]_{365}$ -29.6° (MeOH).

Analysis calc. for $C_{19}H_{25}N_2O_2Cl$

Theory: C, 65.41; H, 7.22; N, 8.03; Cl, 10.16.

Found: C, 65.14; H, 7.21; N, 8.27; Cl, 10.14.

## Example 10

R,R-N-[2-(4-Hydroxyphenyl)-2-hydroxyethyl]-1-methyl-3-(3-hydroxyphenyl)propylaminium chloride

A solution of 19.8 g. of 1-methyl-3-(3-methoxyphenyl)propylamine and 11.5 g. of 4-methoxybenzoylmethyl bromide in 500 ml. of diethyl ether was stirred at room temperature for twenty-four hours. The product was extracted into 10 ml. of water containing 4.8 ml. of concentrated hydrochloric acid. The crystalline solid which precipitated from the

aqueous acid extract was collected and recrystallized from methanol and ethyl acetate to afford 10.5 g. of N-[2-(4-methoxyphenyl)-2-oxoethyl]-1-methyl-3-(3-methoxyphenyl)propylaminium chloride. M.P. 154-156°C.

Analysis calc. for $C_{20}H_{26}NO_3Cl$

Theory: C, 65.90; H, 6.92; N, 3.86; Cl, 9.74.

Found: C, 66.28; H, 7.11; N, 3.78; Cl, 9.88.

A solution of 10 g. of the above-named compound in 100 ml. of forty-eight percent of aqueous hydrobromic acid was heated to reflux and stirred for forty-five minutes. The solution then was cooled and the precipitate which formed was collected by filtration and then dissolved in 80 ml. of water containing 80 ml. of concentrated hydrochloric acid. The precipitated solid was collected by filtration and recrystallized from methanol and ethyl acetate to provide 5.3 g. of N-[2-(4-hydroxyphenyl)-2-oxoethyl]-1-methyl-3-(3-hydroxyphenyl)propylaminium chloride. M.P. 196-198°C. (decomposition).

Reduction of the compound so formed by reaction with hydrogen at a pressure of 40 psi in the presence of 500 mg. of five percent palladium on carbon in 200 ml. of fifty percent aqueous ethanol provided, after crystallization from ethyl acetate and methanol, 5.3 g. of N-[2-(4-hydroxyphenyl)-2-hydroxyethyl]-1-methyl-3-(3-hydroxyphenyl)propylaminium chloride. M.P. 145-146.5°C. (decomposition).

By careful separation of the isomers thus formed, for instance utilizing methods such as high pressure liquid chromatography, followed by resolution of the racemic diastereomers utilizing standard resolution techniques there is provided the compound contemplated by this invention, namely R,R-N-[2-(4-hydroxyphenyl)-2-hydroxyethyl]-1-methyl-3-(3-hydroxyphenyl)propylamine.

Example 11

R,R-N-[2-(3-Hydroxyphenyl)-2-hydroxyethyl]-1-methyl-3-(4-hydroxyphenyl)propylaminium fumarate

According to the general procedure set forth in Example 6, 8.7 g. of R,R-N-[2-(3-benzyloxyphenyl)-2-dichloroacetoxy-1-oxoethyl]-1-methyl-3-(4-benzyloxyphenyl)propylamine was reduced by reaction with 65 ml. of a 1 N solution of diborane in tetrahydrofuran. Upon complete reduction of the amide, as demonstrated by thin layer chromatographic analysis, any excess diborane was destroyed by the dropwise addition to the reaction mixture of 20 ml. of water and 40 ml. of 3 N hydrochloric acid. The acidic reaction mixture then was made alkaline by the addition of 50 ml. of 5 N sodium hydroxide. The product was extracted into ethyl acetate and washed with brine and with water, dried, and the solvent was removed to provide 7.3 g. of R,R-N-[2-(3-benzyloxyphenyl)-2-hydroxyethyl]-1-methyl-3-(4-benzyloxyphenyl)propylamine. The amine so formed was converted to its hydrochloride salt, which crystallized from methanol and ethyl acetate. M.P. 138-141°C. $[\alpha]_D$ -24.9° (MeOH).

Analysis calc. for $C_{32}H_{36}NO_3Cl$

Theory: C, 74.19; H, 7.00; N, 2.70.

Found: C, 74.34; H, 7.06; N, 2.91.

A solution of 4.68 g. of the amine hydrochloride so formed in 120 ml. of methanol containing 25 ml. of ethanol was hydrogenated at a pressure of 60 psi for six hours at 55°C. in the presence of 1.0 g. of five percent palladium on carbon. The product was isolated by filtering the reaction mixture and evaporating the solvent from the filtrate. The residual oil was dissolved in ethyl acetate and made alkaline by the addition of aqueous potassium carbonate. The organic layer was washed with brine, water, and dried. Removal of the solvent by evaporation under reduced pressure provided 2.5 g. of a foam. The foam was dissolved in 600 ml. of ethyl acetate and this solution was diluted with 800 ml. of diethyl ether containing 1.10 g. of fumaric acid. The precipitate which formed was collected by filtration and dried to provide 2.6 g. of R,R-N-[2-(3-hydroxyphenyl)-2-hydroxyethyl]-1-methyl-3-(4-hydroxyphenyl)propylaminium fumarate.

Analysis calc. for $C_{22}H_{27}NO_7$

Theory: C, 63.30; H, 6.52; N, 3.36.

Found: C, 62.02; H, 7.62; N, 3.03

Example 12

R,R-N-(2-Phenyl-2-hydroxyethyl)-1-methyl-3-(4-fluorophenyl)propylamine

To a stirred solution of 30 g. of dl-1-methyl-3-(4-fluorophenyl)propylamine in 300 ml. of

isopropanol was added dropwise to a solution of 24 g. of styrene oxide in isopropanol. Following the addition, the reaction mixture was heated to reflux and stirred for twelve hours. After cooling the solution to room temperature, the solvent was removed by evaporation to provide the product amine as an oil. The oil was dissolved in diethyl ether and to the ethereal solution was added hydrogen chloride. The precipitated salt was collected and recrystallized several times from acetone to provide, as a white solid, R,R-N-(2-phenyl-2-hydroxyethyl)-1-methyl-3-(4-fluorophenyl)propylaminium chloride admixed with the corresponding S,R-isomer. M.P. 141-142°C. The mixture of isomers was evaluated biologically and can be used, as such, in the method of this invention, or alternatively can be further resolved by routine methods.

Example 13

R,R-N-(2-Phenyl-2-hydroxyethyl)-1-methyl-3-(4-aminocarbonylphenyl)propylamine

To a stirred refluxing solution of 12 g. of R-1-methyl-3-(4-aminocarbonylphenyl)propylamine in 100 ml. of ethanol was added dropwise over thirty minutes a solution of 8.3 g. of R-styrene oxide in 25 ml. of ethanol. Following complete addition of the styrene oxide, the reaction mixture was heated at reflux for six hours, and then cooled to room temperature. Removal of the solvent by evaporation under reduced pressure afforded 8.3 g. of the product as a

solid. The solid thus formed was recrystallized three times from methanol to provide 4.5 g. of R,R-N-(2-phenyl-2-hydroxyethyl)-1-methyl-3-(4-aminocarbonylphenyl)propylamine. M.P. 150-152°C.

Analysis calc. for $C_{19}H_{24}N_2O_2$

Theory: C, 73.05; H, 7.74; N, 8.97.

Found: C, 73.02; H, 7.80; N, 8.75.

The amine thus formed was converted to its hydrochloride salt by reaction with hydrogen chloride in diethyl ether to provide R,R-N-(2-phenyl-2-hydroxyethyl)-1-methyl-3-(4-aminocarbonylphenyl)propylaminium chloride. M.P. 256-258°C. $[\alpha]_D$ -16.3°, $[\alpha]_{365}$ -38.8° (MeOH).

Analysis calc. for $C_{19}H_{25}N_2O_2Cl$

Theory: C, 65.41; H, 7.22; N, 8.03.

Found: C, 65.40; H, 7.00; N, 7.90.

## Example 14

R,R-N-[2-(2-Fluorophenyl)-2-hydroxyethyl]-1-methyl-3-(4-aminocarbonylphenyl)propylamine

A solution of 4.0 g. of R-*ortho*-fluorostyrene oxide in 25 ml. of ethanol was added dropwise over thirty minutes to a refluxing solution of 5.0 g. of R-1-methyl-3-(4-aminocarbonylphenyl)propylamine in 100 ml. of ethanol. The reaction mixture was heated at reflux for seven hours following the addition. The hot solution next was filtered and then was cooled to 0°C. and stored at that temperature for twelve hours. The product had crystallized out of solution and was recovered by filtration and recrystallized from fresh

ethanol to provide 2.8 g. of R,R-N-[2-(2-fluorophenyl)-2-hydroxyethyl]-1-methyl-3-(4-aminocarbonylphenyl)propylamine. M.P. 160-162°C. $[\alpha]_D$ -7.1°; $[\alpha]_{365}$ -11.3° (MeOH).

Analysis calc. for $C_{19}H_{23}N_2O_2F$

Theory: C, 69.07; H, 7.02; N, 8.48.

Found: C, 69.02; H, 6.84; N, 8.21.

The amine base so formed was converted to a pharmaceutically acceptable acid addition salt by reaction with hydrogen chloride in diethyl ether to provide R,R-N-[2-(2-fluorophenyl-2-hydroxyethyl]-1-methyl-3-(4-aminocarbonylphenyl)propylaminium chloride. M.P. 228-230°C. $[\alpha]_D$ -14.8°; $[\alpha]_{365}$ -38.6° (MeOH).

Analysis calc. for $C_{19}H_{24}N_2O_2ClF$

Theory: C, 62.38; H, 6.34; N, 7.66.

Found: C, 62.19; H, 6.28; N, 7.55.

## Example 15

R,R-N-(2-Phenyl-2-hydroxyethyl)-1-methyl-3-(4-acetoxyphenyl)propylamine hydrochloride

The hydrochloride salt from Example 6 was converted to the free base by treatment with aqueous sodium carbonate. Extraction with ethyl acetate and evaporation of the solvent under reduced pressure gave R,R-N-(2-phenyl-2-hydroxyethyl)-1-methyl-3-(4-hydroxyphenyl)propylamine, m.p. 136-138°C., $[\alpha]_D$ -13°, $[\alpha]_{365}$ -26.4° (MeOH).

To a solution of 5.0 g. of the free amine thus formed in 300 ml. of tetrahydrofuran, 300 ml. of benzene and 20 ml. of triethylamine was added dropwise with stirring a solution of 4.15 g. of thionyl chloride in 100 ml. of benzene. The reaction mixture was stirred for two-and-one-half hours at 25°C. and washed three times with an aqueous solution of sodium bicarbonate and then twice with water. The organic layer was concentrated under reduced pressure to leave 6.7 g. of R,R-N-[1-methyl-3-(4-hydroxyphenyl)propyl]-5-phenyl-1-oxo-4,5-dihydro-1,2,3-oxathiazole as a brown oil.

A solution of 6.7 g. of the above-named oil in 100 ml. of benzene and 2.5 ml. of pyridine was stirred at room temperature while 1.9 ml. of acetic anhydride was added. The reaction mixture was refluxed for three-and-one-half hours, cooled and poured into 50 ml. of xylene. The solvents were removed under reduced pressure to leave a brown oil which was dissolved in 100 ml. of ethanol and 10 ml. of 3N hydrochloric acid. After stirring for fifteen minutes at room temperature, toluene was added and the solvents were evaporated under reduced pressure. The dark solid was dissolved in hot ethyl acetate and allowed to crystallize. The solid, 4.1 gm., was filtered, dissolved in hot water, treated with decolorizing characoal, filtered, and allowed to cool. Filtration and drying afforded 2.31 g. of R,R-N-(2-phenyl-2-hydroxyethyl)-1-methyl-3-(4-acetoxyphenyl)propylamine hydrochloride, m.p. 173.5-175.5°C., $[\alpha]_D$ -18°, $[\alpha]_{365}$ -51.9° (MeOH).

Analysis calc. for $C_{20}H_{26}ClNO_3$

Theory: C, 66.02; H, 7.20; N, 3.85; Cl, 9.74.

Found : C, 66.30; H, 6.96; N, 3.95; Cl, 9.57.

Example 16

Preparation of 2 mg. Tablets

| | |
|---|---|
| R,R-N-[2-(4-hydroxyphenyl)-2-hydroxyethyl]-1-methyl-3-(4-hydroxyphenyl)propylaminium acetate | 2 mg. |
| Lactose | 98 mg. |
| Corn Starch | 10 mg. |
| Corn Starch Paste | 5 mg. |
| Calcium stearate | 2 mg. |
| Dicalcium phosphate | 28 mg. |

Tablets are formulated by uniformly blending the active drug, corn starch, lactose and dicalcium phosphate until all the ingredients are uniformly admixed. The corn starch paste next is prepared as a 10 percent aqueous paste and it then is added to the mixture and blended to uniformity. The mixture is next blended with the calcium stearate and then compressed into tablets. Tablets containing 4 mg., 6 mg. and the like are formulated in similar fashion.

Example 17

Preparation of Buccal Seal for Transbuccal Administration

| | |
|---|---|
| R,R-N-(2-phenyl-2-hydroxyethyl)-1-methyl-3-(4-aminocarbonylphenyl)propylaminium oxalate | 5 mg. |
| Lactose | 150 mg. |
| Ethanol | 5 ml. |

The active drug and lactose are dissolved in the ethanol and the solution is added to the cavity of a buccal seal made of gums such as guar gum, tragacanth, gum acacia, methyl cellulose and the like. The ethanol is then removed by evaporation to leave a uniform mixture of the active inotropic drug and lactose deposited as a solid in the cavity of the buccal seal. The seal then is administered to a subject and adheres to the inner membrane of the mouth. The active drug is then gradually released.

Example 18

Preparation for Topical Administration

| | |
|---|---|
| R,R-N-(2-phenyl-2-hydroxyethyl)-1-methyl-3-(4-hydroxyethyl)propyl-aminium chloride | 20 mg. |
| Cetyl alcohol | 1000 mg. |
| Sodium lauryl sulfate | 50 mg. |
| Liquid silicone | 600 mg. |
| Methylparaben | 30 mg. |
| Sterile water | 2000 mg. |

The above ingredients are mixed and stirred at about 50°C. and then permitted to congeal. The preparation can be readily applied to the skin in order to effect physical conditioning of the biological system.

Example 19

Preparation for Suppositories

| | |
|---|---|
| R,R-N-[2-(2-fluorophenyl)-2-hydroxyethyl]-1-methyl-3-(3-hydroxyphenyl)-propylamine | 15 mg. |
| Theobroma oil | 1800 mg. |

The above ingredients are blended at a temperature of about 60°C. and then permitted to cool in a tapered mold. Each suppository will weigh about 2 g. and can be administered to a subject about once each day for the treatment of conditions of depressed cardiac contractility.

CLAIMS

1. A compound of the formula (I):

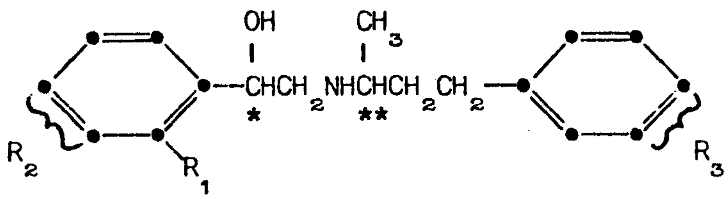

(I)

wherein:

$R_1$ is hydrogen or fluorine;

$R_2$ is hydrogen or hydroxy;

$R_3$ is hydrogen, hydroxy, fluorine, aminocarbonyl, methylaminocarbonyl, methoxycarbonyl or acetoxy;

C* and C** both are asymmetric carbon atoms having the R absolute stereochemical configuration;

with the limitation that at least one of $R_1$ and $R_2$ is hydrogen;

or a pharmaceutically acceptable salt thereof.

2. A compound as claimed in claim 1, wherein $R_2$ is hydroxy.

3. A compound as claimed in claim 2 in which $R_2$ is 4-hydroxy and $R_3$ is 4-hydroxy or 4-aminocarbonyl.

4. R,R-N-[2-(4-Hydroxyphenyl)-2-hydroxyethyl-1-methyl-3-(4-aminocarbonylphenyl)propylamine.

5. R,R-N-[2-(3-Hydroxyphenyl)-2-hydroxyethyl]-1-methyl-3-(4-hydroxyphenyl)propylamine.

6. R,R-N-[2-(4-Hydroxyphenyl)-2-hydroxyethyl]-1-methyl-3-(4-hydroxyphenyl)propylamine.

7. R,R-N-[2-(2-Fluorophenyl)-2-hydroxyethyl]-1-methyl-3-(4-hydroxyphenyl)propylamine.

8. A pharmaceutical formulation comprising a compound of formula (I) as claimed in any one of claims 1 to 7, or a pharmaceutically-acceptable salt-thereof, associated with a pharmaceutically-acceptable carrier therefor.

9. A compound of formula (I) as claimed in any one of claims 1 to 7 for use as an inotropic agent.

10. A process for preparing a compound of the formula (I) as claimed in any one of claims 1 to 7 which process comprises:

a) deprotecting a compound of formula (V):

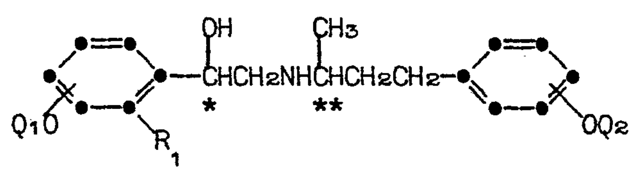

(V)

where $R_1$ is as defined above and $Q_1$ and $Q_2$ are the same or different hydroxy protecting groups, or one of $Q_1$ and $Q_2$ is hydrogen, to form a compound of formula (I) in which $R_2$ and/or $R_3$ is hydroxy;

b) reducing a compound of formula:

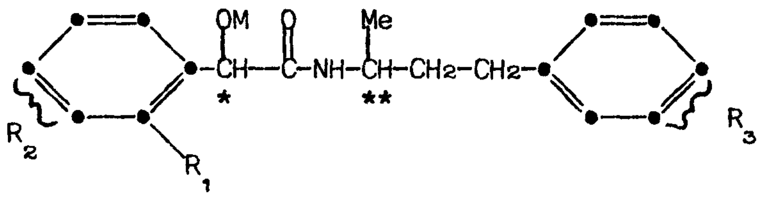

where $R_1$, $R_2$ and $R_3$ are as defined in claim 1, and M is hydrogen or a protecting group, followed in the case where M is not hydrogen by removal of the protecting group by hydrolysis; or

c) reacting a styrene oxide of formula:

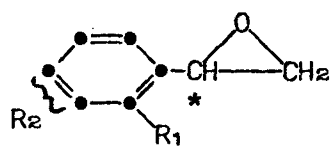

with an optically-active amine of formula (IV)

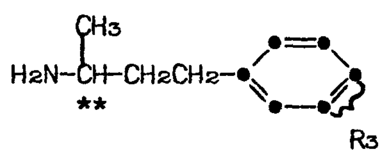 (IV)

where $R_1$, $R_2$ and $R_3$ are as defined in claim 1.

11. An amine of formula (IV) as defined in claim 10.

European Patent Office

# EUROPEAN SEARCH REPORT

Application number

EP 79 301 280.8

## DOCUMENTS CONSIDERED TO BE RELEVANT

| Category | Citation of document with indication, where appropriate, of relevant passages | Relevant to claim |
|---|---|---|
| | DE - B - 1 182 245 (N.V. PHILIPS)<br>* claims 1 and 2; column 2, paragraph 1, formula IIIa, column 6 *<br>-- | 1,2,8<br>11 |
| | DE - A - 1 543 918 (TROPONWERKE)<br>* claim 1; page 2, paragraph 2 *<br>-- | 1 |
| | Chemical Abstracts, Volume 80, Nr. 17, April 29, 1974<br>(COLUMBUS, OHIO, USA)<br>J.Van DIJK et al. "Synthesis of ß-phenylethylamine derivatives. X.N-(Hydroxy- and methoxyaralkyl) derivatives", page 366, column 2, abstract Nr. 95398 Z<br>& Recl. Trav. Chim., Pays-Bas, Volume 92, Nr. 12, 1973, pages 1281 to 1297 (Eng)<br>-- | 1 |
| A | DE - A1 - 2 413 102 (BOEHRINGER)<br>-- | |
| A | DE - A - 2 044 573 (TROPONWERKE)<br>-- | |
| A | FR - A - 1 555 583 (N.V. PHILIPS)<br>---- | |

**CLASSIFICATION OF THE APPLICATION (Int. Cl.)**

C 07 C 91/16
A 61 K 31/135
A 61 K 31/165
A 61 K 31/215
C 07 C 91/30
C 07 C 91/34
C 07 C 93/26
C 07 C 101/42
C 07 C 103/28
C 07 C 103/76

**TECHNICAL FIELDS SEARCHED (Int.Cl.)**

A 61 K 31/135
A 61 K 31/165
A 61 K 31/215
C 07 C 91/16
C 07 C 91/30
C 07 C 91/34
C 07 C 93/26
C 07 C 101/42
C 07 C 103/28
C 07 C 103/76

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| Berlin | 18-10-1979 | KAPTEYN |